# EUROPEAN PATENT SPECIFICATION

(11) **EP 2 779 897 B1**
(45) Date of publication and mention of the grant of the patent: **31.07.2019**
(21) Application number: 12850269.7
(22) Date of filing: 16.11.2012
(51) Int. Cl.: A61B 5/103, G01L 1/24, G01D 5/353, A61B 1/00, A61B 5/11, A61B 5/00, A61B 5/22, G01D 11/30, G01K 11/32

(54) **AN OPTICAL SENSING DEVICE**
OPTISCHE SENSORVORRICHTUNG
DISPOSITIF DE DÉTECTION OPTIQUE

(30) Priority: 16.11.2011 AU 2011904787
(43) Date of publication of application: 24.09.2014
(73) Proprietor: Commonwealth Scientific and Industrial Research Organisation, Campbell, ACT 2612 (AU)
(72) Inventor: ARKWRIGHT, John William, Ryde, New South Wales 2112 (AU); UNDERHILL, Ian, Eagle Heights, Queensland 4217 (AU); BLENMAN, Neil Geoffrey, Carlingford, New South Wales 2118 (AU)
(74) Representative: Barnfather, Karl Jon
(86) International application number: PCT/AU2012/001418
(87) International publication number: WO 2013/071361

(56) References cited:
- EP-A1- 2 331 923
- WO-A1-2006/094353
- WO-A1-2011/048509
- WO-A2-2008/057370
- US-A1- 2008 192 230
- US-A1- 2009 177 095
- US-A1- 2009 177 095
- US-A1- 2012 220 879

## Description

### Field of the Invention

The present invention broadly relates to an optical sensing device.

### Background of the Invention

The human body has many regions in which pressures or forces cause matter to move. For example, the human heart pumps blood through the body. Muscles around the alimentary canal apply a pressure to the canal, which moves food from the mouth into the stomach. Additionally, there are localised regions that exhibit increased muscular tension in their resting state. These regions are commonly referred to as sphincters.

Monitoring pressures motion and muscular tension in the human body can provide important information about the function of the human body and can be used to detect disorders and diseases or can be used to control a recovery from a disease.

Optical devices for monitoring such parameters within a body lumen are now being developed. The optical devices may comprise an optical fibre Bragg grating, which has an optical response that depends on a strain of the Bragg grating. The strain of the Bragg grating may be applied by a "squeezing" force in the vicinity of the Bragg grating and the resultant increase in strain shifts a wavelength of an optical response to a different wavelengths range. However, important information characterising a radial distribution of tensions, effected by muscles, or other properties cannot be provided with known optical devices and there is a need for technological improvement.

Prior patent publication number US 2008/192230 represents the closest prior art and discloses an apparatus for pressure sensing. The apparatus comprises a light guide, a Bragg grating incorporated into the light guide and a moveable wall portion. The moveable wall portion has opposite first and second sides and is being positioned so that a change in pressure at one of the sides relative to a pressure at the other side will move the moveable wall portion. The moveable wall portion is coupled to the Bragg grating so that the movement of the moveable wall portion causes a force on a side of the Bragg grating. The force has a component that is transversal to the Bragg grating and effects a change in strain of the Bragg grating and thereby a change in an optical period of the Bragg grating.

Prior patent publications WO 2008/057230 and US 2009/177095 disclose optical pressure catheters which comprise external grooves arranged to guide optical light guides along the support member.

### Summary of the Invention

The present invention provides an optical sensing device for sensing a pressure or force within a body lumen, the device comprising:
a plurality of optical light guide portions, each optical light guide portion having a sensing region for sensing a pressure or force;
a support for supporting the optical light guide portions, the support being a tubular member and having a plurality of recesses at the sensing regions, the sensing regions having two or more different radial positions around a portion of the support, the optical light guide portions being attached to the support at the sensing regions such that the sensing regions are positioned between attachment regions, the support being rigid at the sensing regions and arranged such that a strain in any one of the sensing regions is not directly influenced by a change in strain of an or the optical light guide portion outside that sensing region;
wherein the optical device is arranged to sense a pressure or force at the two or more different radial positions such that it is possible to sense the pressure or force in a multi-directional manner;
characterised in that each optical light guide portion is positioned around the support and the support comprises at least one groove that is helical and is arranged to guide at least one optical light guide portion around the support.

The optical light guide portions may be optical fibre portions and at least some optical fibre portions may be portions of the same optical fibre. Alternatively, each optical light guide portion may be a portion of a respective optical fibre.

The optical device may have any number of sensing regions at any number of radial orientations that is greater than 1, such as greater than 2, 3, 4, 6, 8, or greater than 10.

In one example the optical sensing device is arranged for sensing the property within a body lumen and may be used to analyse an anorectal function or a function of the oesophagus. For example, the optical sensing device may be arranged to sense muscular contraction within the body lumen, such asymmetric muscular contraction localised to a sphincter region. As two or more sensing regions have differing radial positions, it is typically possible to sense the property in a multi-directional manner, which has significant practical advantages.

In one embodiment the property is a pressure or a force. Alternatively, the property may be any other property of interest, such as a temperature. Further, at least one sensing region may be arranged to sense a first property, such as a pressure, and at least one other sensing region may be arranged to sensing a second property, such as a local temperature.

The optical sensing device typically is arranged such that a force or pressure applied in a direction transversal to at least one optical light guide portion at a respective sensing region is detectable by the optical sensing device.

The support may be arranged such that at least two optical light guide portions are substantially parallel to each other.

In one embodiment some or all of the sensing regions comprise a Bragg grating. At least one of the sensing regions may not comprise a Bragg grating and may be arranged for sensing a property using other means, such as by coherent backscatter techniques or optical frequency domain reflectometry.

Further, at least some sensing regions with Bragg gratings may be arranged to sense a first property, such as a force or pressure, and at of the sensing regions with Bragg gratings may be arranged to sense a second property, such as a local temperature, such that an influence of a change in temperature on the sensing of the first property can be determined.

Each Bragg grating typically is arranged to give a different optical response such that light reflected from each Bragg grating is wavelength division multiplexed. Consequently, it is possible to associate a particular detected pressure change with a respective position. The optical sensing device may be arranged such that the optical response from each Bragg grating can be detected by detecting light that is reflected back from the Bragg gratings. Alternatively, the optical sensing device may be arranged such that the optical responses from the Bragg gratings can be detected by detecting light that is transmitted through the Bragg gratings.

In a variation of this embodiment at least some of the Bragg gratings are substantially identical and give the same response if the strain conditions are the same. Using time or frequency domain reflectometry techniques, the position of a particular Bragg grating may be estimated from a time at which an optical response is received or from an associated beat frequency component.

The optical sensing device typically is arranged for sensing the property in a manner such that the radial and longitudinal position, relative to the axis of the optical sensing device and at which the property is sensed, can be determined.

In one specific embodiment the optical sensing device is arranged for sensing local pressures or forces at sensing regions equipped with Bragg gratings. In this case the optical sensing device is arranged such that the radial and longitudinal positions at which the pressures or forces are sensed can be determined.

The optical sensing device may be arranged such that an applied sideways force results in a local distortion of the Bragg grating, typically in a direction towards the support, which in turn effects a change in strain of the Bragg grating. The change in strain causes a change in an optical response of the Bragg grating and consequently the external force or pressure can be sensed at each location of the Bragg gratings.

The support may comprise a tubular member. The recesses of the support may be provided in the form of through holes.

The optical sensing device may comprise a plurality of optical fibres, such as 2 - 10, typically 2, 4 or 6 optical fibres and each optical fibre may comprise a series of optical fibre portions with of Bragg gratings at sensing regions, such as 10 - 30 Bragg gratings or any other suitable number of Bragg gratings.

The optical fibres typically are spaced apart from each other and positioned in a helical manner around the support such that sensing regions having immediately adjacent radial positions are sensing regions of adjacent optical fibres and not sensing regions of the same optical fibre. The optical fibres may be spaced apart from each other and positioned in a helical manner around the support such that at least some sensing regions have overlapping or substantially identical radial positions.

In one specific embodiment the optical sensing device is arranged for sensing, in a radially resolved manner, external forces or pressures from more than 2, 3, 4, 5 or more radial directions. The optical sensing device typically is arranged to sense a distribution or change in the distribution of external forces or pressures in radial and longitudinal direction such that a 3-dimensional map of the pressure or force distribution around the optical sensing device can be generated.

The optical sensing device may also comprise an outer sleeve, such as an elastic sleeve that may be removable and may have a closed distal end. For example, the optical sensing device may be arranged for insertion into a body lumen and the outer sleeve, typically formed from a biocompatible material, may be replaced after use of the optical sensing device such that the optical sensing device is then suitable for insertion into a body lumen of another patient. The elastic sleeve typically is arranged to cover areas of the sensing device that would otherwise be in contact with the body lumen.

The optical sensing device may comprise an inner tube that typically is positioned within the support and an inflatable balloon which may form a portion of the elastic sleeve. The optical sensing device may be arranged for inflation of the balloon with the body lumen. For example, the optical sensing device may be arranged such that inflation of the balloon stimulates or relaxes muscles at the body lumen. In one specific example the optical sensing device is arranged such that the inflation of the balloon triggers an anorectic function of the human body.

The optical sensing device may comprise a plurality of sensing regions that are each arranged for sensing pressure or force and may comprise moveable wall portions at each sensing region for sensing the pressure or force, the movable wall portions having opposite first and second sides, each moveable wall portion being positioned such that a change in pressure at one of the sides relative to a pressure at the other side will move the moveable wall portion, the moveable wall portions being coupled to respective Bragg gratings such that the movement of one of the moveable wall portion causes a force on the respective Bragg grating resulting in a change in strain of the respective Bragg grating. The moveable wall portions may be wall portions of a tube which may be a tubular diaphragm in which the support and at least a portion of the at least one optical fibre are positioned.

The invention will be more fully understood from the following description of specific embodiments of the invention. The description is provided with reference to the accompanying drawings.

### Brief Description of the Drawings

Figures 1 shows a schematic representation of an optical sensing system according to a specific embodiment of the present invention;
Figures 2 show an image of an optical sensing device according to an embodiment of the present invention;
Figure 3 and 4 show components of the optical sensing device in accordance with specific embodiments of the present invention; and
Figures 5 - 7 show components of a kit for sensing a property in a body lumen according to another specific embodiment of the present invention.

### Detailed Description of Specific Embodiments

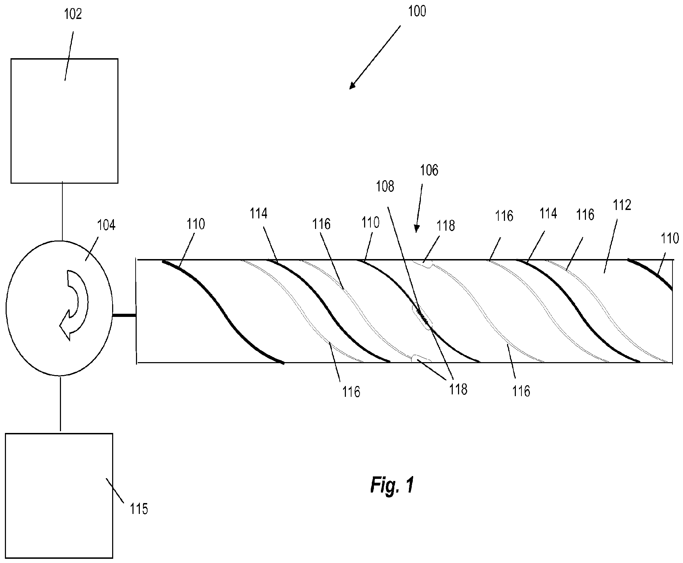

Referring to Figure 1 to 4, an optical sensing system according to a specific embodiment of the present invention is now described. Figure 1 shows a schematic representation of the system 100 comprising a light source 102 which in this embodiment is a broadband light source commonly referred to as a "white" light source even though the light that is emitted by the light source 102 may have any wavelength range.

The light is directed via optical circulator 104 to an optical sensing device 106. In a variation of this embodiment the circulator 104 may be replaced by an optical coupler, an optical splitter or an optical beam splitter. Optical responses from the optical sensing device are directed to an optical analyser 115.

The optical sensing device 106 comprises Bragg gratings 108 incorporated into optical fibres 110. For illustrative purposes the optical sensing device 106 is shown with only one optical fibre 110. Further, the apparatus 106 comprises a support 112 that is provided in the form of a tube. The optical fibres 110 are positioned around the support 112 in a helical manner and located in helical groves 116 of the support 112.

The optical sensing device comprises typically 2, 4, 6, 8, or more optical fibres which are all positioned in helical groves of the support 112 and connected to respective circulators 104. When multiple optical fibres are used, an optical switch may be positioned between the device and the circulator to provide rapid switching between the fibres in order to achieve near real-time interrogation of the sensing regions contained in all of the optical fibres. Alternatively, the plurality of optical fibres may be arranged such that each and every sensing region exhibits a different spectral component such that they may be detected using wavelengths division multiplexing (wdm) techniques, or may have substantially the same spectral components but be arranged such that the distance between each optical fibre and the detector provides a unique distance to each and every sensing region. This allows for unique interrogation of each and every sensing region using time of flight or frequency domain techniques

In this embodiment the support 112 comprises recesses 118. The recesses 118 are provided in the form of holes through walls of the support 112. Portions of the optical fibre 110 cross the recesses 118 and the optical fibres 110 are arranged such that the Bragg gratings 108 are positioned over respective recesses 118. The optical fibres 110 are rigidly attached at attachment regions to the support 112. The attachment regions are located adjacent (over) the recesses 118 such that sensing regions are defined between the attachment regions and at the Bragg gratings 108 positioned over the recesses 118. It is consequently possible to measure the pressures or forces at the sensing regions largely independent from each other and thereby measure a pressure or force distribution.

The function of each sensing region is similar to that disclosed in PCT international application PCT/AU2007/001018. If a force or pressure is applied to the Bragg gratings in a transversal direction of the optical sensing device 106, the optical fibre 110 will be bent, which will effect a change in strain of the Bragg grating 108. For example, if the force or pressure is directed towards the axis of the optical sensing device 106, the optical fibre will be bent inwardly. The change in strain effects a change in an optical property of the Bragg grating and consequently the external force is detectable by detecting the change of the optical property of the Bragg grating.

The optical fibres 110 are positioned around the support 112 in respective helical groves 116. Each optical fibre 110 may comprise a series of Bragg gratings 108, for example series of 10 or 20 or any other suitable number of Bragg gratings. The support 112 and the optical fibres 110 typically are arranged such that sensing regions of adjacent optical fibres are aligned at the same radial orientation relative to the axis of the optical sensing device 106. For example, the optical sensing device 106 may comprise 4 groups of sensing regions, each comprising 10 or 20 sensing regions that are oriented along the axis of the optical sensing device 106. Each group of sensing regions may have a radial orientation that differs by 90 degrees from a radial orientation of an adjacent group sensing regions. Consequently, the optical sensing device 106 is in this example arranged to detect a radial distribution of external forces from 4 orthogonal directions relative to the axis of the optical sensing device 106.

The Bragg grating of at least one of the sensing regions may not be arranged for exposure to the force or pressure, but may be arranged for sensing an influence of a change in local temperature on an optical property of the Bragg grating. Consequently, an influence of a change in local temperature may be determined and forces or pressures sensed at other sensing regions can be corrected accordingly. The optical sensing device 106 typically comprises a plurality of such temperature sensing regions distributed along the optical sensing device 106 (not shown). The structure may have no recesses at the temperature sensing regions such that the optical fibre with Bragg grating cannot move in response to an external force or pressure. Alternatively, the structure may have recesses at the pressure sensing regions, but the optical fibre and an outer sleeve may be spaced apart from each other such that the external force moves the outed sleeve, but not the optical fibre at the temperature sensing region.

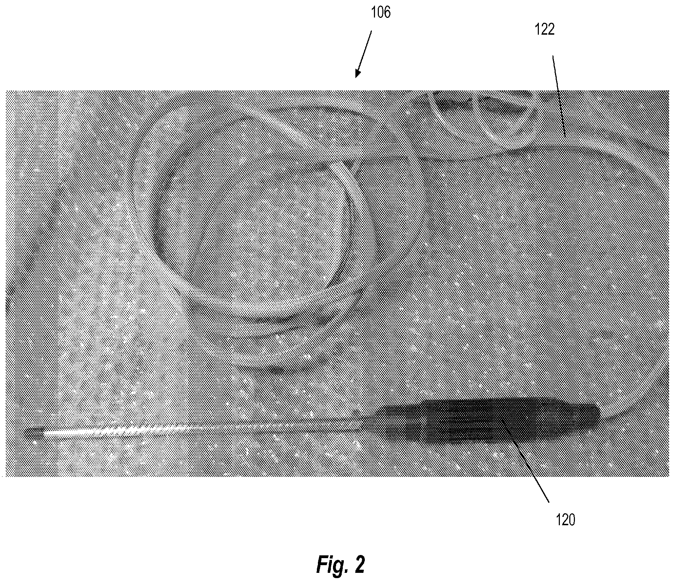

Figure 2 shows an image of the optical sensing device 106. The optical sensing device 106 is in this embodiment arranged for insertion into a body lumen and may be used to monitor an anorectal function of the human body. The optical sensing device 106 as shows in Figure 2 comprises a handle 120 and connecting leads 122 through which the optical fibres 110 are directed and which is coupled to optical circulator 104. The handle or other location may contain one or more optical combiners in order to combine the optical signals from two or more of the discrete fibres of the device.

In a variation of the described embodiments the optical sensing device 106 may also comprise one or more optical fibres that do not have Bragg gratings and/or may be arranged for sensing properties other than pressures or forces. For example, such optical fibres may be arranged to measure a change in temperature, pH, or the presence of luminal or rectal content.

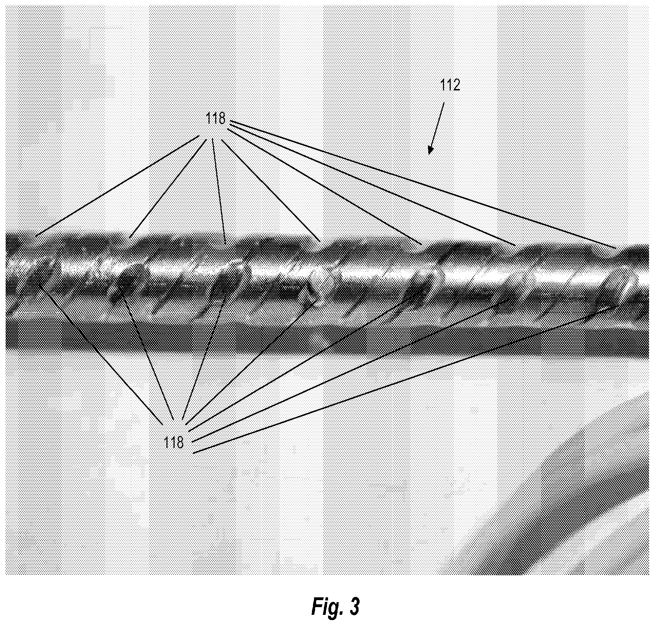

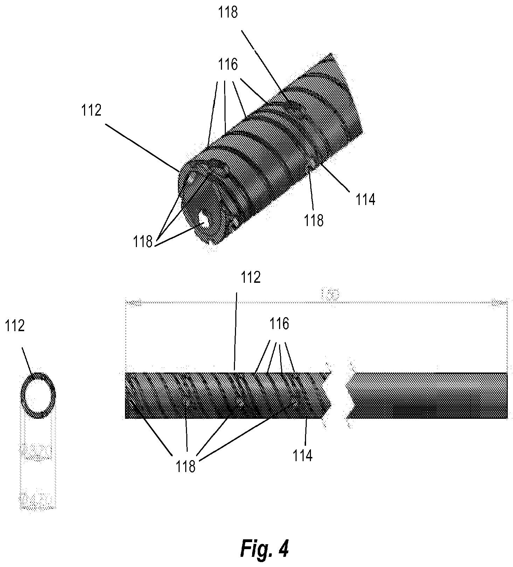

Figures 3 and 4 show portions of the support 112 in more detail. In this embodiment the support 112 comprises a hollow tube formed from a metallic material, such as stainless steel or nitinol, or any suitable plastics or ceramics material that provides adequate mechanical support and or the desired flexibility. The support 112 has a helical through-cut 114 that is parallel with the groves 116 for the optical fibres 110. The helical cut 114 increases the bending flexibility of the optical sensing device 106 while at the same time rigidity in a direction along the groves 116 is largely maintained. Since the optical fibres 110 typically are positioned in parallel with the through-cut 114, an impact of the bending on a strain as experienced by the Bragg gratings 108 is Reduced.

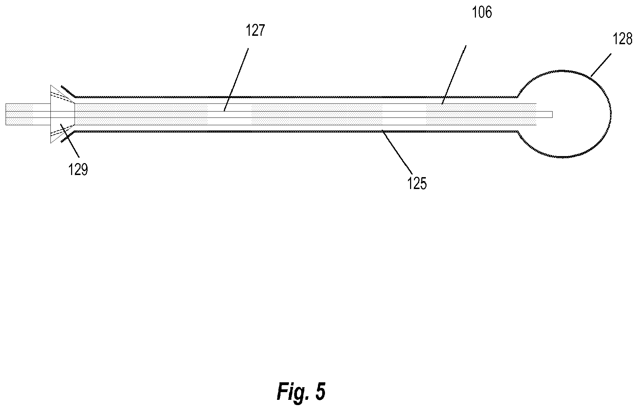

Figure 5 illustrates the optical sensing device 106 positioned in an elastic sleeve 125. The elastic sleeve 125 may be composed of latex or other suitable elastomeric material and is positioned over the optical sensing device 106 to cover areas that would otherwise be in contact with the body lumen during use of the optical sensing device 106. In this embodiment the apparatus 106 also comprises an inner tube 127. Further, the elastic sleeve 125 comprises in this example an inflatable balloon 128 at a distal end an inflation nozzle 129 at the proximal end.

When the optical sensing device is inserted into the body lumen, the balloon 128 may be inflated, via the inner tube 127, to trigger contraction or relaxing of muscles at the body lumen.

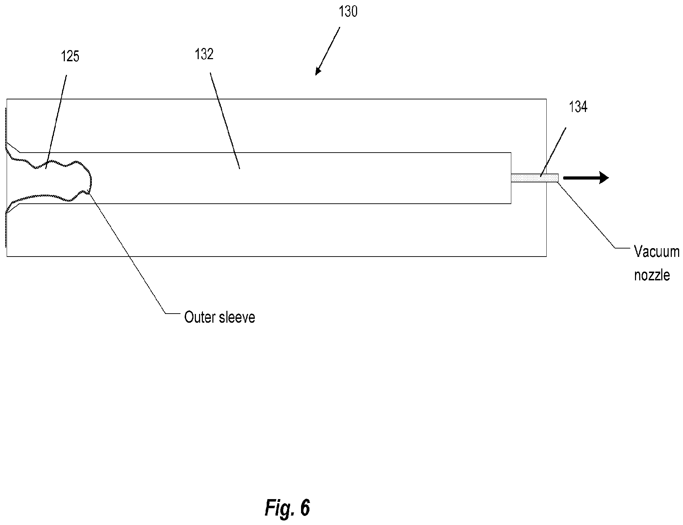

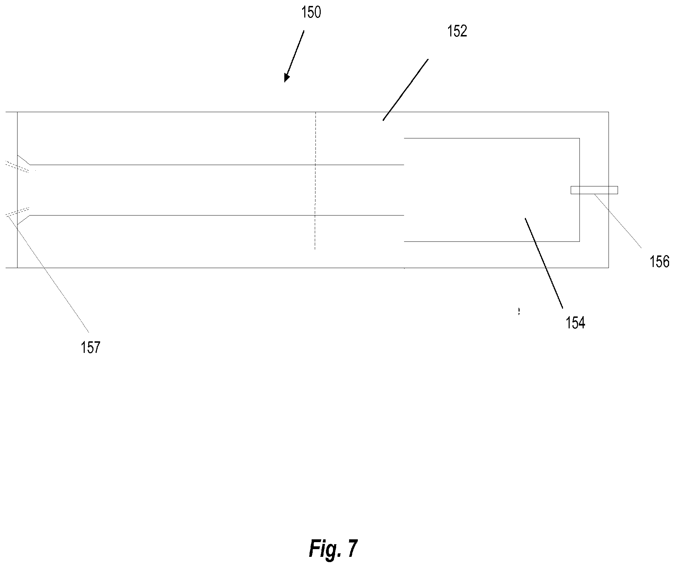

Figures 6 and 7 show components of a kit for sensing a property in a body lumen in accordance with a specific embodiment of the present invention. Figure 6 shows an applicator 130 having a hollow interior portion 132 and vacuum nozzle 134. The elastic sleeve 125 is applied to an open end of the applicator 130. A suction force is generated using a suitable pump (not shown) and the suction force is used to evacuate the interior portion 132 and thereby expand the elastic sleeve 125 until the elastic sleeve 125 is positioned at wall portions of the interior portion 132. The assembled support 112 with optical fibres, over which a protective elastic sleeve may be positioned (such as a further elastic sleeve that may have perforations to allow passage of air), is then inserted into the interior portion 132. An increase of pressure relaxes the elastic sleeve 125 such that the elastic sleeve 125 is positioned over the assembled support 112 with optical fibres 110 (and the protective elastic sleeve if present).

Figure 7 shows a remover 150 for removing the elastic sleeve 125 after use of the optical sensing device 106. The remover 150 comprises a housing 152 having a hollow interior portion 154 that has an exhaust nozzle 156 and a pressure nozzle 157. The remover 150 is proportioned such that the optical sensing device 106 with elastic sleeve 125 can be inserted into the hollow portion 154. Then air pressure is applied to the interior of the optical sensing device 106, or substantially underneath the outer sleeve and between the outer sleeve and the remainder of the device such that the elastic sleeve 125 is ejected into the removable sanitary container, 152 in readiness for sanitary disposal. The optical sensing device 106 without sleeve 125 can then be pulled out of the interior portion 154 and a fresh elastic sleeve 125 can be applied using the applicator 130 such that the device 106 is arranged for reuse. The remover 150 has the significant advantage that the elastic sleeve 125 can be removed from the optical sensing device 106 without the need for touching the outer sleeve 125 by personnel.

In the above-described embodiment each Bragg grating 108 of each optical fibre 110 has a slightly different refractive index variation such that each Bragg grating 108 has an optical response that has a slightly different spectral distribution. The light that is produced by light source 102 and that is directed to the Bragg gratings 108 therefore causes unique responses from the Bragg gratings 108 which are directed via the optical circulator 104 to optical analyser 115 for optical analysis. Such a procedure is commonly referred to as wavelength division multiplexing (WDM) . The Bragg gratings 108 may also effect optical responses which overlap in wavelength or frequency space as long as sufficient information is known about each Bragg grating 108 or its location with respect to the detector to allow the signals to be successfully deconvolved.

As in this embodiment each Bragg grating 108 causes a different response, it is possible to associate a particular response with a radial and longitudinal position relative to the optical sensing device 106 to perform distributed pressure measurements and detect relative pressure differences between the positions of the Bragg gratings 108 in the series. The combined response from the Bragg gratings 108 is wavelength division multiplexed and the optical analyser 114 uses known wavelength division de-multiplexing techniques to identify the responses from the respective grating positions. Suitable software routines are used to determine a pressure or pressure distribution from the optical responses received from the Bragg gratings 108. Pressure measurements typically include calibrating the optical sensing device 106.

In a variation of this embodiment at least some of the Bragg gratings 108 may be identical and consequently, if the strain conditions are the same, their optical response will also be the same. In this case a pulsed light source may be used to guide light to the Bragg gratings 108 and the positions of the Bragg gratings 108 may be estimated from a time at which the responses are received by the optical analyser 115.

In one particular example the reflectivity of each Bragg grating 108 is chosen such that each response has, at the location of the optical analyser 115, approximately the same intensity.

It will be appreciated that in a further variation of this embodiment the apparatus may be arranged such that responses from respective Bragg gratings can be analysed by receiving light that is transmitted through the Bragg gratings 108. For example, in this case the optical sensing device 106 typically is arranged such that light is guided from the light source 102 through the Bragg gratings 108 and then directly to the optical analyser 115.

## Claims

1. An optical sensing device for sensing a pressure or force within a body lumen, the device comprising:
a plurality of optical light guide portions (110), each optical light guide portion having a sensing region for sensing a pressure or force;
a support (112) for supporting the optical light guide portions, the support being a tubular member and having a plurality of recesses (118) at the sensing regions, the sensing regions having two or more different radial positions around a portion of the support, the optical light guide portions being attached to the support at the sensing regions such that the sensing regions are positioned between attachment regions, the support being rigid at the sensing regions and arranged such that a strain in any one of the sensing regions is not directly influenced by a change in strain of an or the optical light guide portion outside that sensing region;
wherein the optical device is arranged to sense a pressure or force at the two or more different radial positions such that it is possible to sense the pressure or force in a multi-directional manner;
**characterised in that** the support (112) comprises at least one groove that is helical and is arranged to guide at least one optical light guide portion (110) around the support (112) and each optical light guide portion (110) is positioned around the support (112) in a respective groove.

2. The optical sensing device of claim 1 having sensing regions at more than two angular orientations relative to an axis of the device.

3. The optical sensing device of claim 1 or 2 wherein the light guide portions (110) are optical fibre portions, wherein at least some optical light guide portions comprise Bragg gratings (108)at sensing regions.

4. The optical sensing device of any one of the preceding claims wherein the optical sensing device is arranged for sensing the pressure or force in a manner such that a radial and longitudinal position can be determined.

5. The optical sensing device of any one of the preceding claims wherein the recesses are provided in the form of through holes.

6. The optical sensing device of claim 3 or claim 4 any when dependent on claim 3 wherein the optical sensing device is arranged such that an applied sideways force results in a local distortion of one or more Bragg gratings (108), which in turn effects a change in strain of the Bragg grating.

7. The optical sensing device of any one of the preceding claims comprising a plurality of optical fibres (110) and each optical fibre comprising a plurality of optical light guide portions with Bragg gratings (108).

8. The optical sensing device of any one of the preceding claims wherein the optical light guide portions (110) are positioned spaced apart from each other in a helical manner around the support (112) such that sensing regions having immediately adjacent radial positions are sensing regions of adjacent optical fibres and not sensing regions of the same optical fibre.

9. The optical sensing device of any one of the preceding claims where at least one of the following apply:
a) the optical sensing device comprises an outer elastic sleeve (128) that is removable;
b) the optical sensing device comprises an inflatable balloon (128), and outer elastic sleeve that is removable;
c) at least some sensing regions are arranged to sense a force or pressure and at least one sensing region with at least one sensing region is arranged to sense a local temperature such that an influence of a change in temperature on the sensing of the force of pressure can be determined.

## Patentansprüche

1. Optische Sensorvorrichtung zum Erfassen eines Drucks oder einer Kraft innerhalb eines Körperlumens, wobei die Vorrichtung folgendes umfasst:
eine Vielzahl von optischen Lichtleiterabschnitten (110), wobei jeder optische Lichtleiterabschnitt einen Sensorbereich zur Erfassung eines Drucks oder einer Kraft aufweist;
einen Träger (112) zur Halterung der optischen Lichtleiterabschnitte, wobei der Träger ein röhrenförmiges Element mit einer Vielzahl von Aussparungen (118) an den Sensorbereichen ist, wobei die Sensorbereiche zwei oder mehr unterschiedliche radiale Positionen um einen Abschnitt des Trägers herum einnehmen, wobei die optischen Lichtleiterabschnitte am Träger derart an den Sensorbereichen befestigt sind, dass die Sensorbereiche zwischen Befestigungsbereichen positioniert sind, wobei der Träger an den Sensorbereichen steif und derart ausgelegt ist, dass eine Dehnung in einem der Sensorbereiche nicht direkt von einer Änderung der Dehnung eines bzw. des optischen Lichtleiterabschnitts außerhalb dieses Sensorbereichs beeinflusst wird;
wobei die optische Vorrichtung zur Erfassung eines Drucks oder einer Kraft an den zwei oder mehreren unterschiedlichen radialen Positionen ausgelegt ist, um dadurch eine multidirektionale Erfassung des Drucks oder der Kraft zu ermöglichen;
**dadurch gekennzeichnet, dass** der Träger (112) mindestens eine Nut aufweist, die spiralförmig verläuft und ausgelegt ist, um mindestens einen optischen Lichtleiterabschnitt (110) um den Träger (112) herum zu führen, und jeder optische Lichtleiterabschnitt (110) um den Träger (112) herum in einer entsprechenden Nut verläuft.

2. Optische Sensorvorrichtung nach Anspruch 1 mit Sensorbereichen in mehr als zwei Winkelausrichtungen relativ zu einer Achse der Vorrichtung.

3. Optische Sensorvorrichtung nach Anspruch 1 oder 2, bei der die Lichtleiterabschnitte (110) Glasfaserabschnitte sind, wobei mindestens einige optische Lichtleiterabschnitte in Sensorbereichen Bragg-Gitter (108) umfassen.

4. Optische Sensorvorrichtung nach einem der vorhergehenden Ansprüche, bei der die optische Sensorvorrichtung zum Erfassen des Drucks oder der Kraft derart angeordnet ist, um die Bestimmung von Radial- und Längsposition zu ermöglichen.

5. Optische Sensorvorrichtung nach einem der vorhergehenden Ansprüche, bei der die Aussparungen in Form von Durchgangsbohrungen vorgesehen sind.

6. Optische Sensorvorrichtung nach Anspruch 3 oder Anspruch 4 bei Rückbezug auf Anspruch 3, bei der die optische Sensorvorrichtung derart angeordnet ist, dass eine angelegte seitliche Kraft zu einer lokalen Verzerrung eines oder mehrerer Bragg-Gitter(s) (108) führt, was wiederum eine Änderung der Dehnung des Bragg-Gitters bewirkt.

7. Optische Sensorvorrichtung nach einem der vorhergehenden Ansprüche, umfassend eine Vielzahl von optischen Fasern (110), wobei jede optische Faser eine Vielzahl von optischen Lichtleiterabschnitten mit Bragg-Gittern (108) umfasst.

8. Optische Sensorvorrichtung nach einem der vorhergehenden Ansprüche, bei der die optischen Lichtleiterabschnitte (110) derart im Abstand voneinander spiralförmig um den Träger (112) herum angeordnet sind, dass Sensorbereiche mit direkt benachbarten radialen Positionen Sensorbereiche benachbarter optischer Fasern und nicht Sensorbereiche derselben optischen Faser sind.

9. Optische Sensorvorrichtung nach einem der vorstehenden Ansprüche, bei der mindestens eine der nachstehenden Bedingungen erfüllt ist:
a) die optische Sensorvorrichtung umfasst eine äußere elastische Hülse (128), die abnehmbar ist;
b) die optische Sensorvorrichtung umfasst einen aufblasbaren Ballon (128) und eine äußere elastische Hülse, die abnehmbar ist;
c) mindestens einige Sensorbereiche sind zur Erfassung einer Kraft oder eines Drucks und mindestens eines Sensorbereichs angeordnet, wobei mindestens ein Sensorbereich zur Erfassung einer lokalen Temperatur angeordnet ist, um die Bestimmung eines Einflusses einer Temperaturänderung auf das Erfassen der Kraft oder des Drucks ermöglicht wird.

## Revendications

1. Un dispositif de détection optique pour détecter une pression ou une force dans une lumière d'un organisme, le dispositif comprenant :
une pluralité de parties de guide d'onde optique (110), chaque partie de guide d'onde optique ayant une région de détection pour détecter une pression ou une force ;
un support (112) pour supporter les parties de guide d'onde optique, le support étant un membre tubulaire et ayant une pluralité de cavités (118) dans les régions de détection, les régions de détection ayant deux positions radiales différentes ou plus autour d'une partie du support, les parties de guide d'onde optique étant attachées au support dans les régions de détection de telle sorte que les régions de détection soient positionnées entre les régions d'attachement, le support étant rigide dans les régions d'attachement et disposées de telle sorte qu'une pression s'exerçant sur l'une quelconque des régions de détection n'est pas directement influencée par la pression sur une ou la partie de guide d'onde optique en-dehors de la région de détection ;
dans lequel le dispositif optique est disposé pour détecter une pression ou une force aux deux positions radiales différentes ou plus de sorte qu'il est possible de détecter la pression ou la force d'une manière multidirectionnelle ;
**caractérisé en ce que** le support (112) comprend au moins une cannelure qui est hélicoïdale et est disposée pour guider au moins une partie de guide d'onde optique (110) autour du support (112) et chaque partie de guide d'onde optique (110) est positionnée autour du support (112) dans une cannelure respective.

2. Dispositif de détection optique de la revendication 1 ayant des régions de détection dans plus de deux orientations angulaires par rapport à un axe du dispositif.

3. Dispositif de détection optique de la revendication 1 ou 2 dans lequel les parties de guide d'onde (110) sont des parties de fibre optique, dans lequel au moins certaines des parties de guide d'onde optique comprennent des réseaux de diffraction de Bragg (108) dans les régions de détection.

4. Dispositif de détection optique de l'une quelconque des revendications précédentes dans lequel le dispositif de détection optique est disposé pour détecter la pression ou la force d'une manière telle qu'il est possible de déterminer une position radiale et longitudinale.

5. Dispositif de détection optique de l'une quelconque des revendications précédentes dans lequel les cavités sont fournies sous la forme de trous percés.

6. Dispositif de détection optique de la revendication 3 ou la revendication 4 lorsqu'elle dépend de la revendication 3 dans lequel le dispositif de détection optique est disposé de telle sorte qu'une force appliquée latéralement entraîne une distorsion locale d'un ou plusieurs réseaux de diffraction de Bragg (108), ce qui à son tour provoque un changement de la contrainte exercée sur le réseau de diffraction de Bragg.

7. Dispositif de détection optique de l'une quelconque des revendications précédentes comprenant une pluralité de fibres optiques (110) et chaque fibre optique comprenant une pluralité de parties de guide d'onde optique ayant des réseaux de diffraction de Bragg.

8. Dispositif de détection optique de l'une quelconque des revendications précédentes dans lequel les parties de guide d'onde optique (110) sont espacées l'une de l'autre de manière hélicoïdale autour du support (112) de telle sorte que les régions de détection ayant les positions radiales immédiatement adjacentes soient les régions de détection de fibres optiques adjacentes et ne soient pas des régions de détection de la même fibre optique.

9. Dispositif de détection optique de l'une quelconque des revendications précédentes sur lequel au moins un des points suivants s'applique :
a) le dispositif de détection optique comprend une gaine extérieure élastique (128) qui est détachable ;
b) le dispositif de détection optique comprend un ballonnet gonflable (128), et une gaine extérieure élastique qui est détachable ;
c) au moins certaines des régions de détection sont disposées pour détecter une force ou une pression et au moins une région de détection avec au moins une région de détection est disposée pour détecter une température locale de telle sorte qu'une influence d'un changement de température sur la détection de la force ou de la pression puisse être déterminée.
